# EUROPEAN PATENT APPLICATION

(11) **EP 4 471 122 A1**
(43) Date of publication of application: **04.12.2024**
(21) Application number: 22923640.1
(22) Date of filing: 28.12.2022
(51) Int. Cl.: C12N 1/21, C12P 13/08, C12R 1/15

(54) **RECOMBINANT MICROORGANISM, METHOD FOR CONSTRUCTING SAME AND USE THEREOF**

(30) Priority: 27.01.2022 CN 202210101407
(71) Applicant: Langfang Meihua Biotechnology Development Co., Ltd, Langfang City, Hebei 065001 (CN)
(72) Inventor: KANG, Pei, Langfang City, Hebei 065001 (CN); WANG, Chen, Langfang City, Hebei 065001 (CN); GONG, Weibo, Langfang City, Hebei 065001 (CN); HE, Jun, Langfang City, Hebei 065001 (CN); LI, Yan, Langfang City, Hebei 065001 (CN)
(74) Representative: CH Kilger Anwaltspartnerschaft mbB
(86) International application number: PCT/CN2022/142939
(87) International publication number: WO 2023/142855

(57) **Abstract**

The present invention relates to the technical field of microbial engineering. Specifically disclosed are a recombinant microorganism, a method for constructing same and use thereof. According to the present invention, by means of constructing a phosphate acetyltransferase-inactivated strain and applying the strain to the production of threonine, the threonine-producing ability of the strain is remarkably improved, and the strain has a remarkably increased production of threonine as compared to an unmodified strain. Combined with attenuated expression or inactivation of acetate kinase, HTH-type transcriptional regulator and the like, as well as improved activity of pyruvate carboxylase and enzymes involved in a threonine synthesis-related pathway, the production of threonine is further improved. The described modifications can be used in the fermentative production of threonine and have relatively good application value.

## Description

### Technical Field

The present invention relates to the technical field of microbial engineering, and specifically disclosed are a recombinant microorganism, a method for constructing same and use thereof.

### Background Art

Threonine (β-hydroxy-α-aminobutyric acid) has a molecular formula of C₄H₉NO₃ with a relative molecular mass of 119.12, and is an essential amino acid, mainly used in medicine, chemical reagents, food fortifiers, feed additives, etc.

Corynebacterium glutamatum is an important threonine producing strain. In Corynebacterium glutamatum, oxaloacetate is converted into threonine through a five-step reaction catalyzed by aspartate kinase (encoded by lysC), aspartate semialdehyde dehydrogenase (encoded by asd), homoserine dehydrogenase (encoded by horn), homoserine kinase (encoded by thrB) and threonine synthase (encoded by thrC). At present, reports on the production of threonine by Corynebacterium glutamatum mainly focus on its synthetic pathway, but there are few reports on the supply of threonine precursors and the overflow of pyruvate during the synthesis of threonine.

### Summary of the Invention

An objective of the present invention is to provide a recombinant microorganism for producing threonine by inactivating phosphate acetyltransferase. The present invention further provides a method to engineering the microorganism and use of it.

At present, the metabolic engineering modification on the synthesis of threonine by Corynebacterium glutamatum mainly focuses on the synthesis pathway from oxaloacetate to threonine. Pyruvate, as an important intermediate metabolite in the microbial metabolic network, mainly enters the tricarboxylic acid cycle to provide energy and precursors for bacterial growth. However, when the upstream and downstream metabolic pathways are unbalanced, metabolic overflow of pyruvate will occur, resulting in waste of pyruvate. Oxaloacetate , the precursor of threonine, can be generated from pyruvate catalyzed by pyruvate carboxylase, and can also be generated by a series of enzyme-catalyzed reactions after pyruvate enters the tricarboxylic acid cycle. The present invention discovers that the flow of pyruvate to oxaloacetate, can be improved by reducing the metabolic overflow of pyruvate, thereby promoting the synthesis of threonine, during the metabolic engineering of Corynebacterium glutamicum to produce threonine , Compared to other methods of reducing the metabolic overflow of pyruvate, the effect of reducing or losing the activity of phosphate acetyltransferase is obviously better. Reducing or losing the activity of phosphate acetyltransferase can effectively reduce the metabolic overflow of pyruvate, and improve the supply of threonine synthesis precursors, thereby significantly improving the threonine synthesis ability of strains.

In order to achieve the object of the present invention, in first aspect, the present invention provides a modified Corynebacterium microorganism having reduction or loss the activity of phosphate acetyltransferase compared to the unmodified microorganism and having improved threonine-production compared to the unmodified microorganism.

Preferably, phosphate acetyltransferase has a reference sequence number of NP_601948.1 on NCBI, or an amino acid sequence with 90% similarity and equivalent function thereto.

Further, reduction or loss the activity of phosphate acetyltransferase in the microorganism is achieved by reducing the expression of the gene encoding phosphate acetyltransferase or knocking out an endogenous gene encoding phosphate acetyltransferase.

Mutagenesis, site-directed mutation or homologous recombination can be used to reduce the expression of the gene encoding phosphate acetyltransferase or knock off the endogenous gene encoding phosphate acetyltransferase.

Further, the microorganism has improved the activity of the pyruvate carboxylase and/or obtained feedback-resistant pyruvate carboxylase compared to the unmodified microorganism.

Preferably, pyruvate carboxylase has a reference sequence number of WP_011013816.1 on NCBI, or an amino acid sequence with 90% similarity and equivalent function thereto.

Further, the activity of either or both of the following enzymes (1) to (2) is reduced or lost in the microorganism compared to the unmodified microorganism:
(1) acetate kinase; and
(2) HTH-type transcriptional regulator.

Preferably, acetate kinase and HTH-type transcriptional regulator have the reference sequence numbers of WP_003862874.1 and WP_003859703.1 on NCBI, respectively, or amino acid sequences with 90% similarity and equivalent function thereto.

Further, the microorganism has improved activity of the enzymes involved in a threonine synthesis pathway and/or deregulated the enzymes involved in the threonine synthesis pathway compared to the unmodified microorganism in vivo; wherein the enzyme involved in the threonine synthesis pathway is selected from at least one of aspartate kinase, homoserine dehydrogenase, and threonine synthase.

Preferably, aspartate kinase, homoserine dehydrogenase and threonine synthase have the reference sequence numbers of WP_003855724.1, WP_003855724.1, and WP_011014964.1 on NCBI, respectively, or amino acid sequences with 90% similarity and equivalent function thereto.

Preferably, the microorganism is any of the following (1) to (4):
(1) a microorganism with reduced or lost activity of phosphate acetyltransferase and improved activity of and/or deregulation of aspartate kinase, homoserine dehydrogenase, threonine synthase and/or pyruvate carboxylase;
(2) a microorganism with reduced or lost activity of phosphate acetyltransferase and/or acetate kinase and improved activity of and/or deregulation of aspartate kinase, homoserine dehydrogenase, threonine synthase and/or pyruvate carboxylase;
(3) a microorganism with reduced or lost activity of phosphate acetyltransferase and/or HTH-type transcriptional regulator, and improved activity of and/or deregulation of aspartate kinase, homoserine dehydrogenase, threonine synthase and/or pyruvate carboxylase;
(4) a microorganism with reduced or lost activity of at least one of phosphate acetyltransferase, acetate kinase and HTH-type transcriptional regulator, and improved activity of and/or deregulation of aspartate kinase, homoserine dehydrogenase, threonine synthase and/or pyruvate carboxylase.

The enhancement of the activity of the above enzymes is achieved by any one selected from the following 1) to 6), or an optional combination thereof:
1) enhancement by introduction of a plasmid having the gene encoding the enzyme;
2) enhancement by increasing the copy number of the gene encoding the enzyme on the chromosome;
3) enhancement by altering the promoter sequence of the gene encoding the enzyme on the chromosome;
4) enhancement by operably linking a strong promoter to the gene encoding the enzyme;
5) enhancement by altering the amino acid sequence of the enzyme; and
6) enhancement by altering the nucleotide sequence encoding the enzyme.

Preferably, the enhancement of the activity of the enzyme is achieved by replacing the original promoter of the gene encoding the enzyme with a stronger promoter with greater activity, and/or by mutating the start codon of the gene to ATG.

The strong promoter comprises Psod or PcspB.

The nucleotide sequences of promoters Psod and PcspB are shown as SEQ ID NOs. 1 and 2, respectively.

Preferably, improved activity of pyruvate carboxylase, aspartate kinase, and threonine synthase is achieved by replacing their original promoter with a Psod promoter;

Improved activity of homoserine dehydrogenase is achieved by replacing its original promoter with a PcspB promoter.

The deregulation described above is preferably achieved by the following mutations: the feedback-resistant pyruvate carboxylase is achieved by mutating the gene encoding pyruvate carboxylase, so that the encoded pyruvate carboxylase has a P458S mutation;
the feedback-resistant aspartate kinase is achieved by mutating the gene encoding aspartate kinase, so that the encoded aspartate kinase has a T311I mutation.
the feedback-resistant homoserine dehydrogenase is achieved by mutating the gene encoding homoserine dehydrogenase, so that the encoded homoserine dehydrogenase has a G378E mutation.

Preferably, the microorganism of the present invention is Corynebacterium glutamicum. Corynebacterium glutamatum includes ATCC13032, ATCC13870, ATCC13869, ATCC21799, ATCC21831, ATCC14067, ATCC13287, etc. (see NCBI Corunebacterium glutamicum evolutionary tree https://www.ncbi.nlm.nih.gov/genome/469), more preferably Corynebacterium glutamatum ATCC 13032.

In a second aspect, the present invention provides a method for constructing a threonine-producing strain, the method comprising:
A. attenuating a gene encoding phosphate acetyltransferase in amino acid producing Corynebacterium to obtain a gene-attenuated strain, wherein, the attenuating includes knocking out or reducing the expression of the gene encoding phosphate acetyltransferase; and/or
B. enhancing the activity of pyruvate carboxylase and/or deregulationof pyruvate carboxylase; and/or
C. reducing or losing the activity of any one or more of the following enzymes (1)-(2): (1) acetate kinase;
   and (2) HTH-type transcriptional regulator; and/or
D. enhancing the activity of an enzyme involved in the threonine synthesis pathway and/or deregulation the enzyme involved in the threonine synthesis pathway, wherein the enzyme involved in the threonine synthesis pathway is selected from at least one of aspartate kinase, homoserine dehydrogenase, and threonine synthase.

The routine to enhance the activity is selected from the following 1) to 6), or an optional combination thereof:
1) enhancement by introduction of a plasmid having the gene encoding the enzyme;
2) enhancement by increasing the copy number of the gene encoding the enzyme on the chromosome;
3) enhancement by altering the promoter sequence of the gene encoding the enzyme on the chromosome;
4) enhancement by operably linking a strong promoter to the gene encoding the enzyme;
5) enhancement by altering the amino acid sequence of the enzyme; and
6) enhancement by altering tthe nucleotide sequence encoding the enzyme;
and/or, the reduction or loss of the activity is achieved by reducing the expression or knocking out the gene encoding the enzyme.

In a third aspect, the present invention provides a method for producing threonine, the method comprises the steps of:
a) cultivating the microorganism to obtain a culture of the microorganism;
b) collecting threonine from the culture obtained in step a).

In a fourth aspect, the present invention provides the use of reducing or losing the enzyme activity of phosphate acetyltransferase in threonine fermentation or increasing threonine production.

Preferably, the reduction or loss the activity of phosphate acetyltransferase in the microorganism is achieved by knocking out or reducing the expression of the gene encoding phosphate acetyltransferase .

Further, the production of threonine is increased by inactivation of phosphate acetyltransferase in amino acid producing Corynebacterium.

Preferably, the Corynebacterium of the present invention is Corynebacterium glutamicum. Corynebacterium glutamatum includes ATCC13032, ATCC13870, ATCC13869, ATCC21799, ATCC21831, ATCC14067, ATCC13287, etc. (see NCBI Corunebacterium glutamicum evolutionary tree https://www.ncbi.nlm.nih.gov/genome/469), more preferably Corynebacterium glutamatum ATCC 13032.

In a fifth aspect, the present invention provides the use of the modified Corynebacterium microorganism or a threonine-producing strain constructed according to the above method in threonine fermentation or increasing threonine production.

The above-mentioned modification methods of related strains, including gene enhancement and attenuation, are all modification methods known to those skilled in the art, see Man Zaiwei. Systemic pathway engineering modification of Corynebacterium crenatum to produce L-arginine with high yield [D]. Jiangnan University, 2016; Cui Yi. Metabolic engineering modification of Corynebacterium glutamicum to produce L-leucine [D]. Tianjin University of Science and Technology.; Xu Guodong. Construction of L-isoleucine producing strain and optimization of fermentation conditions. Tianjin University of Science and Technology, 2015.

The beneficial effects of the present invention are as follows: The present invention reduces the metabolic overflow of pyruvate by inactivating phosphate acetyltransferase, thus reducing overflow metabolites, which reducing the waste of pyruvate, and making more pyruvate flow to oxaloacetate, the precursor of threonine synthesis, so that the threonine-producing ability of the strain is remarkably improved, and the strain has a remarkably increased yield of threonine as compared to an unmodified strain. Combined with attenuated expression or inactivation of acetate kinase, HTH-type transcriptional regulator and the like, as well as improved activity of pyruvate carboxylase and enzymes involved in a threonine synthesis pathway, the yield of threonine is further improved. The described modifications can be used in the fermentative production of threonine and have relatively good application value.

### Detailed Description of Embodiments

The following examples are intended to illustrate the present invention but are not intended to limit the scope of the present invention.

Information on a protein and a gene encoding the protein according to the following examples is as follows:
phosphate acetyltransferase, coding gene pta, NCBI number: cg3048, Cgl2753, NCgl2657.
aspartate kinase, coding gene lysC, NCBI number: cg0306, Cgl0251, NCgl0247.
homoserine dehydrogenase, coding gene horn, NCBI number: cg1337, Cgl1183, NCgl1136.
threonine synthase, coding gene thrC, NCBI number: cg2437, Cgl2220, NCgl2139.
pyruvate carboxylase, coding gene pyc, NCBI number: cg0791, Cgl0689, NCgl0659.
acetate kinase, coding gene ackA, NCBI number: cg3047, Cgl2752, NCgl2656.
HTH-type transcriptional regulator RamB, coding gene ramB, NCBI number: cg0444, Cgl0369, NCgl0358.

### Example 1 Construction of plasmids for genome modification of strains

### 1. Construction of the plasmid pK18mobsacB-P_{sod}-lysC^{g1a-T311I} for enhancing expression of aspartate kinase

The upstream homologous arm up was obtained by PCR amplification with P21/P22 primer pair using ATCC13032 genome as template, the promoter fragment Psod was obtained by PCR amplification with P23/P24 primer pair, lysC^{g1a-T311I} was obtained by PCR amplification with P25/P26 primer pair, and the downstream homologous arm dn was obtained by PCR amplification with P27/P28 primer pair. The up-Psod fragment was obtained by fusion PCR with P21/P24 primer pair using up and Psod as templates. The full-length fragment up-Psod-lysC^{g1a-T311I}-dn was obtained by fusion PCR with P21/P28 primer pair using up-Psod, lysC^{g1a-T311I} and dn as templates. pK18mobsacB was digested with BamHI/HindIII. Enzyme-digested up-Psod-lysC^{g1a-T311I}-dn and pK18mobsacB were assembled using a seamless cloning kit and transformed into Trans1 T1 competent cells to obtain the recombinant plasmid pK18mobsacB-P_{sod}-*lysC*^{g1a-T311I}.

### 2. Construction of plasmid pK18mobsacB-P_{cspB}-hom^{G378E} for improved expression of homoserine dehydrogenase

The plasmid construction method was referred to above 1, and the primers used were P29, P30, P31, P32, P33, P34, P35, and P36.

### 3. Construction of the plasmid pk18mobsacB-Psod-thrC^{g1a} for enhancing expression of threonine synthase

The plasmid construction method was referred to above 1, and the primers used were P37, P38, P39, P40, P41, and P42.

### 4. Construction of the plasmid pK18mobsacB-Psod-pyc^{P458S} for enhancing expression of pyruvate carboxylase

The plasmid construction method was referred to above 1, and the primers used were P13, P14, P15, P16, P17, P18, P19, and P20.

### 5. Construction of plasmid pK18mobsacB-△pta for inactivating phosphate acetyltransferase

The upstream homologous arm up was obtained by PCR amplification with P67/P68 primer pair using ATCC13032 genome as template; the downstream homologous arm dn was obtained by PCR amplification with P69/P70 primer pair; and the fragment up-dn was obtained by fusion PCR with P67/P70 primer pair using up and dn as templates. pK18mobsacB was digested with BamHI/HindIII. Enzyme-digested up-dn and pK18mobsacB were assembled using a seamless cloning kit and transformed into Trans1 T1 competent cells to obtain the recombinant plasmid pk18mobsacB-△pta.

### 6. Construction of plasmid pk18mobsacB-△ackA for inactivating acetate kinase

The plasmid construction method was referred to above 5, and the primers used were P165, P166, P167, and P168.

### 7. Construction of plasmid pk18mobsacB-△pta-△ackA for co-inactivating phosphate acetyltransferase and acetate kinase

The plasmid construction method was referred to above 5, and the primers used were pta-ackAup1, pta-ackAup2q, pta-ackAdn1q, and P168.

### 8. Construction of plasmid pk18mobsacB-ramB^{a1g} for attenuating HTH-type transcriptional regulator RamB

The plasmid construction method was referred to above 5, and the primers used were P115, P116, P117, and P118.

### 9. Construction of plasmid pk18mobsacB-△ramB for inactivating HTH transcriptional regulator

The plasmid construction method was referred to above 5, and the primers used were P119, P120, P121, and P122.

The primers used in the above plasmid construction process are shown in Table 1.

**Table 1 Primer sequences**

| Name | Sequence |
|---|---|
| P13 | AATTCGAGCTCGGTACCCGGGGATCCTGACAGTTGCTGATCTGGCT |
| P14 | CCCGGAATAATTGGCAGCTATAGAGTAATTATTCCTTTCA |
| P15 | TGAAAGGAATAATTACTCTATAGCTGCCAATTATTCCGGG |
| P16 | GAAGATGTGTGAGTCGACACGGGTAAAAAATCCTTTCGTA |
| P17 | TACGAAAGGATTTTTTACCCGTGTCGACTCACACATCTTC |
| P18 | GGTGGAGCCTGAAGGAGGTGCGAGTGATCGGCAATGAATCCGG |
| P19 | CCGGATTCATTGCCGATCACTCGCACCTCCTTCAGGCTCCACC |
| P20 | GTAAAACGACGGCCAGTGCCAAGCTTCGCGGCAGACGGAGTCTGGG |
| P21 | AATTCGAGCTCGGTACCCGGGGATCCAGCGACAGGACAAGCACTGG |
| P22 | CCCGGAATAATTGGCAGCTATGTGCACCTTTCGATCTACG |
| P23 | CGTAGATCGAAAGGTGCACATAGCTGCCAATTATTCCGGG |
| P24 | TTTCTGTACGACCAGGGCCATGGGTAAAAAATCCTTTCGTA |
| P25 | TACGAAAGGATTTTTTACCCATGGCCCTGGTCGTACAGAAA |
| P26 | TCGGAACGAGGGCAGGTGAAGGTGATGTCGGTGGTGCCGTCT |
| P27 | AGACGGCACCACCGACATCACCTTCACCTGCCCTCGTTCCGA |
| P28 | GTAAAACGACGGCCAGTGCCAAGCTTAGCCTGGTAAGAGGAAACGT |
| P29 | AATTCGAGCTCGGTACCCGGGGATCCCTGCGGGCAGATCCTTTTGA |
| P30 | ATTTCTTTATAAACGCAGGTCATATCTACCAAAACTACGC |
| P31 | GCGTAGTTTTGGTAGATATGACCTGCGTTTATAAAGAAAT |
| P32 | GTATATCTCCTTCTGCAGGAATAGGTATCGAAAGACGAAA |
| P33 | TTTCGTCTTTCGATACCTATTCCTGCAGAAGGAGATATAC |
| P34 | TAGCCAATTCAGCCAAAACCCCCACGCGATCTTCCACATCC |
| P35 | GGATGTGGAAGATCGCGTGGGGGTTTTGGCTGAATTGGCTA |
| P36 | GTAAAACGACGGCCAGTGCCAAGCTTGCTGGCTCTTGCCGTCGATA |
| P37 | ATTCGAGCTCGGTACCCGGGGATCCGCCGTTGATCATTGTTCTTCA |
| P38 | CCCGGAATAATTGGCAGCTAGGATATAACCCTATCCCAAG |
| P39 | CTTGGGATAGGGTTATATCCTAGCTGCCAATTATTCCGGG |
| P40 | ACGCGTCGAAATGTAGTCCATGGGTAAAAAATCCTTTCGTA |
| P41 | TACGAAAGGATTTTTTACCCATGGACTACATTTCGACGCGT |
| P42 | GTAAAACGACGGCCAGTGCCAAGCTTGAATACGCGGATTCCCTCGC |
| P67 | AGCTCGGTACCCGGGGATCCTGTCCAACTGCGGTGATT |
| P68 | GGTAGACAAGCAAGGCAACAGGCAAATGTGTTTATCTTCC |
| P69 | GGAAGATAAACACATTTGCCTGTTGCCTTGCTTGTCTACC |
| P70 | CAATACGGAGCGGTTACAAAGCTTGGCACTGGCCGTCG |
| P115 | CATGATTACGAATTCGAGCTCGGTACCCGGGGATCCCGTGCGTGGATTGTCAG CA |
| P116 | GATCACGCTATAGTTGCGCCGTGGGAAAGACATATGTGGG |
| P117 | CCCACATATGTCTTTCCCACGGCGCAACTATAGCGTGATC |
| P118 | TCACGACGTTGTAAAACGACGGCCAGTGCCAAGCTTCACGGTCGGGATTTCTA ACAG |
| P119 | CATGATTACGAATTCGAGCTCGGTACCCGGGGATCCAGTAGACACCTCGAACG CTAC |
| P120 | GATCACGCTATAGTTGCGCCGAAAAGGAGCTTGCTTTACGAC |
| P121 | GTCGTAAAGCAAGCTCCTTTTCGGCGCAACTATAGCGTGATC |
| P122 | TCACGACGTTGTAAAACGACGGCCAGTGCCAAGCTTCACGGTCGGGATTTCTA ACAG |
| P165 | CGAGCTCGGTACCCGGGGATCCACCCGGGTGTGGCGCGCAAGAAGATGCCAG |
| P166 | TAAATGTTGTACGCGGACCAGAACAAGATTCCGCCGTGGACCACGC |
| P167 | GGCGGAATCTTGTTCTGGTCCGCGTACAACATTTACATACACC |
| P168 | GTAAAACGACGGCCAGTGCCAAGCTTAGCAAGGTGTTAGAGCAAATTTTCG |
| pta-ackAup1 | GAATTCGAGCTCGGTACCCGGGGATCCAACTTGTAACCGCTCCGTAT |
| pta-ackAup2 q | GGTGTATGTAAATGTTGTACGCGGACCAGTGCTTGCCTTGCTTGTCTA |
| pta-ackAdn1 q | TAGACAAGCAAGGCAAGCACTGGTCCGCGTACAACATTTACATACACC |

### Example 2 Construction of a genome-modified strain

### 1. Construction of a strain with improved expression of aspartate kinase

ATCC13032 competent cells were prepared according to the classic method of Corynebacterium glutamicum (C. glutamicum Handbook, Chapter 23). The competent cells were transformed with the recombinant plasmid pK18mobsacB-P_{sod}-*lysC*^{g1a-T311I} by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The sequence of interest was amplified by PCR and analyzed by nucleotide sequencing, and the obtained mutant strain of interest was named SMCT121. Compared to ATCC13032 strain, in this strain, the start codon of lysC gene was mutated from GTG to ATG, threonine encoded at amino acid position 311 was mutated to isoleucine, and the promoter of lysC gene was replaced with Psod promoter.

### 2. Construction of a strain with improved expression of homoserine dehydrogenase

The strain construction method was referred to the above 1. SMCT121 is used as the original strain, and the plasmid pK18mobsacB-P_{cspB}-*hom*^{G378E} was introduced into the strain to perform modification for enhancing the expression of homoserine dehydrogenase. The obtained modified strain was named SMCT122. Compared to the original strain SMCT121, the horn gene of this strain was mutated, resulting in the G378E mutation in its encoded protein, and the promoter of the horn gene was replaced with PcspB promoter.

### 3. Construction of a strain with threonine synthase overexpression

The strain construction method was referred to the above 1. SMCT122 is used as the original strain, and the plasmid pk18mobsacB-Psod-thrC^{g1a} was introduced into the strain to perform modification for enhancing the expression of threonine synthase. The obtained modified strain was named SMCT123. Compared to the original strain SMCT122, the start codon of the thrC gene of the strain was mutated from GTG to ATG, and the promoter of the thrC gene was replaced with Psod promoter.

### 4. Construction of a strain with improved expression of pyruvate carboxylase

The strain construction method was referred to the above 1. SMCT123 is used as the original strain, and the plasmid pK18mobsacB-Psod-pyc^{P458S} was introduced into the strain to perform modification for enhancing the expression of pyruvate carboxylase. The obtained modified strain was named SMCT124. Compared to the original strain SMCT123, the pyc gene of this strain was mutated, resulting in the P458S mutation in its encoded protein, and the promoter of the pyc gene was replaced with Psod promoter.

### 5. Construction of a strain with inactivated acetate kinase

The strain construction method was referred to the above 1. SMCT124 is used as the original strain, and the plasmid pk18mobsacB-△ackA was introduced into the strain to perform modification for inactivating acetate kinase. The obtained modified strain was named SMCT125. Compared to the original strain SMCT124, the ackA of this strain was knocked out.

### 6. Construction of a strain with attenuated HTH-type transcriptional regulator

The strain construction method was referred to the above 1. SMCT124 is used as the original strain, and the plasmid pk18mobsacB-ramB^{a1g} was introduced into the strain to perform modification for attenuating ramB. The obtained modified strain was named SMCT126. Compared to the original strain SMCT124, the start codon of the ramB gene of this strain was mutated to GTG.

### 7. Construction of a strain with inactivated HTH-type transcriptional regulator

The strain construction method was referred to the above 1. SMCT124 and SMCT125 were used as original strains, and pk18mobsacB-△ramB was introduced into the above original strains to perform modification for inactivating ramB, respectively. The obtained modified strains were named SMCT127 and SMCT128. Compared to their corresponding original strains, ramB was knocked out in these two strains.

### 8. Construction of a strain with inactivated phosphate acetyltransferase

The strain construction method was referred to the above 1. SMCT124, SMCT125, SMCT126, SMCT127 and SMCT128 were used as original strains, and the pk18mobsacB-△pta or pk18mobsacB-△pta-△ackA plasmid was used to perform modification for inactivating phosphate acetyltransferase. The obtained modified strains were named SMCT129, SMCT130, SMCT131, SMCT132 and SMCT133. Compared to their corresponding original strains, the pta gene of these strains was knocked out.

The genotype information of the strains obtained above is shown in Table 2.

**Table 2 Genotype information of strains**

| Strain Name | Genotype |
|---|---|
| SMCT121 | ATCC13032, P_{sod}-*lysC*^{g1a-T311I} |
| SMCT122 | ATCC13032, P_{sod}-*lysC*^{g1a-T311I}, P_{cspB}-*hom*^{G378E} |
| SMCT123 | ATCC13032, P_{sod}-*lysC*^{g1a-T311I}, P_{cspB}-*hom*^{G378E}, P_{sod}*-thrC*^{g1a} |
| SMCT124 | ATCC13032, P_{sod}-*lysC*^{g1aT311I}, P_{cspB}-*hom*^{G378E}, P_{sod}-*thrC*^{g1a}, P_{sod}-*pyc*^{P458S} |
| SMCT125 | ATCC13032, P_{sod}-*lysC*^{g1a-T311I}, P_{cspB}-*hom*^{G378E}, P_{sod}*-thrC*^{g1a}, P_{sod}-*pyc*^{P458S}, Δ*ackA* |
| SMCT126 | ATCC13032, P_{sod}-*lysC*^{g1a-T311I}, P_{cspB}-*hom*^{G378E}, P_{sod}-*thrC*^{g1a}, P_{sod}-*pyc*^{P458S}, *ramB*^{a1g} |
| SMCT127 | ATCC13032, P_{sod}-*lysC*^{g1a-T311I}, P_{cspB}-*hom*^{G378E}, P_{sod}*-thrC*^{g1a}, P_{sod}-*pyc*^{P458S}, Δ*ramB* |
| SMCT128 | ATCC13032, P_{sod}-*lysC*^{g1a-T311I}, P_{cspB}-*hom*^{G378E}, P_{sod}-*thrC*^{g1a}, P_{sod}-*pyc*^{P458S}, Δ*ackA*, Δ*ramB* |
| SMCT129 | ATCC13032, P_{sod}-*lysC*^{g1a-T311I}, P_{cspB}-*hom*^{G378E}, P_{sod}*-thrC*^{g1a}, P_{sod}-*pyc*^{P458S}, Δ*pta* |
| SMCT130 | ATCC13032, P_{sod}-*lysC*^{g1a-T311I}, P_{cspB}-*hom*^{G378E}, P_{sod}*-thrC*^{g1a}, P_{sod}-*pyc*^{P458S}, Δ*ackA*,Δ*pta* |
| SMCT131 | ATCC13032, P_{sod}-*lysC*^{g1a-T311I}, P_{cspB}-*hom*^{G378E}, P_{sod}-*thrC*^{g1a}, P_{sod}-*pyc*^{P458S}, *ramB*^{a1g}, Δ*pta* |
| SMCT132 | ATCC13032, P_{sod}-*lysC*^{g1a-T311I}, P_{cspB}-*hom*^{G378E}, P_{sod}*-thrC*^{g1a}, P_{sod}-*pyc*^{P458S}, Δ*ramB*, Δ*pta* |
| SMCT133 | ATCC13032, P_{sod}-*lysC*^{g1a-T311I}, P_{cspB}-*hom*^{G378E}, P_{sod}-*thrC*^{g1a}, P_{sod}-*pyc*^{P458S}, Δ*ackA*, *ΔramB,* Δ*pta* |

### Example 3 Shake flask fermentation verification of strains

Each strain constructed in Example 2 was validated by shake flask fermentation as follows:

### 1. Medium

Seed activation medium: BHI 3.7%, agar 2%, pH 7.
Seed medium: Peptone 5/L, yeast extract 5 g/L, sodium chloride 10 g/L, ammonium sulfate 16 g/L, urea 8 g/L, potassium dihydrogen phosphate 10.4 g/L, dipotassium hydrogen phosphate 21.4 g/L, biotin 5 mg/L, magnesium sulfate 3 g/L. Glucose 50 g/L, pH 7.2.
Fermentation medium: corn steep liquor 50 mL/L, glucose 30 g/L, ammonium sulfate 4 g/L, MOPS 30 g/L, potassium dihydrogen phosphate 10 g/L, urea 20 g/L, biotin 10 mg/L, magnesium sulfate 6 g/L, ferrous sulfate 1 g/L, VB1•HCl 40 mg/L, calcium pantothenate 50 mg/L, nicotinamide 40 mg/L, manganese sulfate 1 g/L, zinc sulfate 20 mg/L, copper sulfate 20 mg/L, pH 7.2.

### 2. Production of L-threonine by shake flask fermentation with engineered strain

(1) Seed culture: 1 loop of seed of strains SMCT121, SMCT122, SMCT123, SMCT124, SMCT125, SMCT126, SMCT127, SMCT128, SMCT129, SMCT130, SMCT131, SMCT132 and SMCT133 on the slant culture medium was picked and inoculated into a 500 mL Erlenmeyer flask containing 20 mL of seed culture medium, and cultured at 30°C and 220 r/min for 16 h to obtain a seed broth.
(2) Fermentation culture: 2 mL of seed liquid was inoculated into a 500 mL Erlenmeyer flask containing 20 mL of fermentation medium and cultured at 33°C and 220 r/min under shaking for 24 h to obtain a fermentation broth.
(3) 1 mL of fermentation broth was taken and centrifuged (12000 rpm, 2 min), and the supernatant was collected. L-threonine in the fermentation broth of modified strain and control strain were detected by HPLC.

The fermentation results of the strains with preliminary threonine synthesis ability are shown in Table 3.

**Table 3 Fermentation test results (1)**

| Strain number | OD₅₆₂ | L-threonine (g/L) |
|---|---|---|
| SMCT121 | 23 | 1.2 |
| SMCT122 | 23 | 2.4 |
| SMCT123 | 23 | 3.0 |

As can be seen from Table 3, after the aspartate kinase was modified in the wild strain ATCC13032, the threonine of the engineered strain was initially accumulated. With the improved expression of enzymes (homoserine dehydrogenase and threonine synthase) in the threonine synthesis pathway, the threonine production was further improved, and 3.0 g/L of threonine could be accumulated.

The threonine accumulation in the further engineered strains including modified phosphate acetyltransferase coding gene is shown in Table 4.

**Table 4 Fermentation results (2)**

| Strain number | OD₅₆₂ | L-threonine (g/L) | Strain number | OD₅₆₂ | L-threonine (g/L) |
|---|---|---|---|---|---|
| SMCT124 | 23 | 3.6 | SMCT129 | 23 | 4.3 |
| SMCT125 | 23 | 4.2 | SMCT130 | 23 | 5.3 |
| SMCT126 | 23 | 4.0 | SMCT131 | 23 | 5.0 |
| SMCT127 | 23 | 4.2 | SMCT132 | 23 | 5.3 |
| SMCT128 | 23 | 5.0 | SMCT133 | 23 | 6.5 |

As can be seen from Table 4, the threonine production of the strains after inactivating the pta gene to optimize the carbon metabolic flow and reduce carbon loss is improved to varying degrees, among which the threonine production of SMCT133 is increased by 30% compared to the control strain SMCT128, indicating that more carbon flow to threonine synthesis after the overflow metabolic flux is reduced in the terminal pathway of the engineered strain with improved threonine synthesis pathway Moreover, different combinations of inactivation of the pta and modifications of the pyc (encoding pyruvate carboxylase), ackA (encoding acetate kinase), ramB (encoding HTH transcriptional regulator), lysC (encoding aspartate kinase), horn (encoding homoserine dehydrogenase), thrC (encoding threonine synthase) and the like can all further improve the threonine yield.

Although the present invention has been described in detail above with general descriptions and specific embodiments, it is obvious to those skilled in the art that some modifications or improvements may be made based on the present invention. Therefore, these modifications or improvements made without departing from the spirit of the present invention belong to the scope of protection claimed by the present invention.

## Claims

1. A modified microorganism from the genus *Corynebacterium,* wherein the modified microorganism has its phosphate acetyltransferase activity reduced or lost as compared to the unmodified microorganism, and has an improved threonine-producing ability as compared to the unmodified microorganism.

2. The modified microorganism of claim 1, wherein the activity of phosphate acetyltransferase in the modified microorganism is reduced or lost by reducing the expression of a gene encoding phosphate acetyltransferase or by knocking out an endogenous gene encoding phosphate acetyltransferase.

3. The modified microorganism of claim 2, wherein the reducing the expression of a gene encoding phosphate acetyltransferase or the knocking out an endogenous gene encoding phosphate acetyltransferase is performed by mutagenesis, site-directed mutation, or homologous recombination.

4. The modified microorganism of claim 1, wherein the modified microorganism has improved pyruvate carboxylase activity and/or feedback-resistant pyruvate carboxylase as compared to the unmodified microorganism.

5. The modified microorganism of claim 1 or 4, wherein the activity of either one or both of (1) acetate kinase and (2) HTH-type transcriptional regulator in the modified microorganism is reduced or lost as compared to the unmodified microorganism.

6. The modified microorganism of any one of claims 1 to 5, wherein the modified microorganism has improved activity of an enzyme involved in the threonine synthesis pathway and/or deregulated enzyme involved in the threonine synthesis pathway as compared to the unmodified microorganism;
wherein the enzyme involved in the threonine synthesis pathway is at least one selected from aspartate kinase, homoserine dehydrogenase, and threonine synthase.

7. The modified microorganism of claim 6, which is any of the following (1) to (4):
(1) a microorganism with reduced or lost activity of phosphate acetyltransferase; and improved activity and/or deregulated aspartate kinase, homoserine dehydrogenase, threonine synthase, and/or pyruvate carboxylase;
(2) a microorganism with reduced or lost activity of phosphate acetyltransferase and/or acetate kinase; and improved activity and/or deregulated aspartate kinase, homoserine dehydrogenase, threonine synthase, and/or pyruvate carboxylase;
(3) a microorganism with reduced or lost activity of phosphate acetyltransferase and/or HTH-type transcriptional regulator; and improved activity and/or deregulated aspartate kinase, homoserine dehydrogenase, threonine synthase, and/or pyruvate carboxylase;
(4) a microorganism with reduced or lost activity of at least one of phosphate acetyltransferase, acetate kinase, and HTH-type transcriptional regulator; and improved activity and/or deregulated aspartate kinase, homoserine dehydrogenase, threonine synthase, and/or pyruvate carboxylase;
preferably, the improved activity of the enzyme is achieved by any one of or any combination of the following 1) to 6):
1) introducing a plasmid carrying the gene encoding the enzyme;
2) increasing the copy number of the gene encoding the enzyme in the chromosome;
3) altering the promoter sequence of the gene encoding the enzyme in the chromosome;
4) operably linking a strong promoter to the gene encoding the enzyme;
5) altering the amino acid sequence of the enzyme; and
6) altering the nucleotide sequence encoding the enzyme.

8. The modified microorganism of any one of claims 1 to 7, which is *Corynebacterium glutamicum.*

9. A method for constructing a threonine-producing strain, comprising:
A) attenuating a gene encoding phosphate acetyltransferase in *Corynebacterium* species, which has an amino acid-producing ability, to obtain an attenuated strain, wherein the attenuating includes knocking out or reducing the expression of the gene encoding phosphate acetyltransferase; and/or
B) enhancing the activity of pyruvate carboxylase and/or obtaining feedback-resistant pyruvate carboxylase; and/or
C) reducing or losing the activity of either one or both of (1) acetate kinase and (2) HTH transcriptional regulator;
and/or
D) enhancing the activity of an enzyme involved in the threonine synthesis pathway and/or deregulating the enzyme involved in the threonine synthesis pathway, wherein the enzyme involved in the threonine synthesis pathway is at least one selected from aspartate kinase, homoserine dehydrogenase, and threonine synthase;
wherein the activity of the enzyme is improved by any one of or any combination of the following 1) to 6):
1) introducing a plasmid carrying the gene encoding the enzyme;
2) increasing the copy number of the gene encoding the enzyme in the chromosome;
3) altering the promoter sequence of the gene encoding the enzyme in the chromosome;
4) operably linking a strong promoter to the gene encoding the enzyme;
5) altering the amino acid sequence of the enzyme; and
6) altering the nucleotide sequence encoding the enzyme;
and/or, the reducing or losing the activity is achieved by reducing the expression of the gene encoding the enzyme or knocking out the gene encoding the enzyme.

10. The method of claim 9, wherein the *Corynebacterium* species is *Corynebacterium glutamicum.*

11. A method for producing threonine, comprising the following steps:
a) culturing the engineered microorganism of any one of claims 1-8 or the threonine-producing strain constructed by the method of claim 9 or 10, to obtain a culture of the modified microorganism or the threonine-producing strain;
b) collecting threonine from the culture obtained in step a).


Sequence listing
<110>LANGFANG MEIHUA BIOTECHNOLOGY DEVELOPMENT CO., LTD.
<120>RECOMBINANT MICROORGANISM, METHOD FOR CONSTRUCTING SAME AND USE THEREOF
<130> KHP211124467.8
<160> 51
<170> SIPOSequenceListing 1.0
<210> 1
<211> 192
<212> DNA
<213> Artificial Sequence
<400> 1
tagctgccaa ttattccggg cttgtgaccc gctacccgat aaataggtcg gctgaaaaat 60
ttcgttgcaa tatcaacaaa aaggcctatc attgggaggt gtcgcaccaa gtacttttgc 120
gaagcgccat ctgacggatt ttcaaaagat gtatatgctc ggtgcggaaa cctacgaaag 180
gattttttac cc 192
<210> 2
<211> 260
<212> DNA
<213> Artificial Sequence
<400> 2

acctgcgttt ataaagaaat gtaaacgtga tcggatcgat ataaaagaaa cagtttgtac 60
tcaggtttga agcattttct ccaattcgcc tggcaaaaat ctcaattgtc gcttacagtt 120
tttctcaacg acaggctgct aagctgctag ttcggtggcc tagtgagtgg cgtttacttg 180
gataaaagta atcccatgtc gtgatcagcc attttgggtt gtttccatag catccaaagg 240
tttcgtcttt cgatacctat 260
<210> 3
<211> 46
<212> DNA
<213> Artificial Sequence
<400> 3
aattcgagct cggtacccgg ggatcctgac agttgctgat ctggct 46
<210> 4
<211> 40
<212> DNA
<213> Artificial Sequence
<400> 4
cccggaataa ttggcagcta tagagtaatt attcctttca 40
<210> 5
<211> 40
<212> DNA
<213> Artificial Sequence
<400> 5
tgaaaggaat aattactcta tagctgccaa ttattccggg 40
<210> 6
<211> 40
<212> DNA
<213> Artificial Sequence
<400> 6
gaagatgtgt gagtcgacac gggtaaaaaa tcctttcgta 40
<210> 7
<211> 40
<212> DNA
<213> Artificial Sequence
<400> 7
tacgaaagga ttttttaccc gtgtcgactc acacatcttc 40
<210> 8
<211> 43
<212> DNA
<213> Artificial Sequence
<400> 8
ggtggagcct gaaggaggtg cgagtgatcg gcaatgaatc cgg 43
<210> 9
<211> 43
<212> DNA
<213> Artificial Sequence

<400>  9
ccggattcat tgccgatcac tcgcacctcc ttcaggctcc acc                43
<210>  10
<211>  46
<212>  DNA
<213>  Artificial Sequence
<400>  10
gtaaaacgac ggccagtgcc aagcttcgcg gcagacggag tctggg             46
<210>  11
<211>  46
<212>  DNA
<213>  Artificial Sequence
<400>  11
aattcgagct cggtacccgg ggatccagcg acaggacaag cactgg             46
<210>  12
<211>  40
<212>  DNA
<213>  Artificial Sequence
<400>  12
cccggaataa ttggcagcta tgtgcacctt tcgatctacg                    40
<210>  13
<211>  40
<212>  DNA
<213>  Artificial Sequence
<400>  13
cgtagatcga aaggtgcaca tagctgccaa ttattccggg                    40
<210>  14
<211>  41
<212>  DNA
<213>  Artificial Sequence
<400>  14
tttctgtacg accagggcca tgggtaaaaa atcctttcgt a                  41
<210>  15
<211>  41
<212>  DNA
<213>  Artificial Sequence
<400>  15
tacgaaagga ttttttaccc atggccctgg tcgtacagaa a                  41
<210>  16
<211>  42
<212>  DNA
<213>  Artificial Sequence
<400>  16
tcggaacgag ggcaggtgaa ggtgatgtcg gtggtgccgt ct                 42
<210>  17

<211>  42
<212>  DNA
<213>  Artificial Sequence
<400>  17
agacggcacc accgacatca ccttcacctg ccctcgttcc ga                          42
<210>  18
<211>  46
<212>  DNA
<213>  Artificial Sequence
<400>  18
gtaaaacgac ggccagtgcc aagcttagcc tggtaagagg aaacgt                      46
<210>  19
<211>  46
<212>  DNA
<213>  Artificial Sequence
<400>  19
aattcgagct cggtacccgg ggatccctgc gggcagatcc ttttga                      46
<210>  20
<211>  40
<212>  DNA
<213>  Artificial Sequence
<400>  20
atttctttat aaacgcaggt catatctacc aaaactacgc                            40
<210>  21
<211>  40
<212>  DNA
<213>  Artificial Sequence
<400>  21
gcgtagtttt ggtagatatg acctgcgttt ataaagaaat                            40
<210>  22
<211>  40
<212>  DNA
<213>  Artificial Sequence
<400>  22
gtatatctcc ttctgcagga ataggtatcg aaagacgaaa                            40
<210>  23
<211>  40
<212>  DNA
<213>  Artificial Sequence
<400>  23
tttcgtcttt cgatacctat tcctgcagaa ggagatatac                            40
<210>  24
<211>  41
<212>  DNA
<213>  Artificial Sequence

<400> 24
tagccaattc agccaaaacc cccacgcgat cttccacatc c 41
<210> 25
<211> 41
<212> DNA
<213> Artificial Sequence
<400> 25
ggatgtggaa gatcgcgtgg gggttttggc tgaattggct a 41
<210> 26
<211> 46
<212> DNA
<213> Artificial Sequence
<400> 26
gtaaaacgac ggccagtgcc aagcttgctg gctcttgccg tcgata 46
<210> 27
<211> 46
<212> DNA
<213> Artificial Sequence
<400> 27
attcgagctc ggtacccggg gatccgccgt tgatcattgt tcttca 46
<210> 28
<211> 40
<212> DNA
<213> Artificial Sequence
<400> 28
cccggaataa ttggcagcta ggatataacc ctatcccaag 40
<210> 29
<211> 40
<212> DNA
<213> Artificial Sequence
<400> 29
cttgggatag ggttatatcc tagctgccaa ttattccggg 40
<210> 30
<211> 41
<212> DNA
<213> Artificial Sequence
<400> 30
acgcgtcgaa atgtagtcca tgggtaaaaa atcctttcgt a 41
<210> 31
<211> 41
<212> DNA
<213> Artificial Sequence
<400> 31
tacgaaagga ttttttaccc atggactaca tttcgacgcg t 41
<210> 32

<211> 46
<212> DNA
<213> Artificial Sequence
<400> 32
gtaaaacgac ggccagtgcc aagcttgaat acgcggattc cctcgc 46
<210> 33
<211> 38
<212> DNA
<213> Artificial Sequence
<400> 33
agctcggtac ccggggatcc tgtccaactg cggtgatt 38
<210> 34
<211> 40
<212> DNA
<213> Artificial Sequence
<400> 34
ggtagacaag caaggcaaca ggcaaatgtg tttatcttcc 40
<210> 35
<211> 40
<212> DNA
<213> Artificial Sequence
<400> 35
ggaagataaa cacatttgcc tgttgccttg cttgtctacc 40
<210> 36
<211> 38
<212> DNA
<213> Artificial Sequence
<400> 36
caatacggag cggttacaaa gcttggcact ggccgtcg 38
<210> 37
<211> 55
<212> DNA
<213> Artificial Sequence
<400> 37
catgattacg aattcgagct cggtacccgg ggatcccgtg cgtggattgt cagca 55
<210> 38
<211> 40
<212> DNA
<213> Artificial Sequence
<400> 38
gatcacgcta tagttgcgcc gtgggaaaga catatgtggg 40
<210> 39
<211> 40
<212> DNA
<213> Artificial Sequence

<400> 39
cccacatatg tctttcccac ggcgcaacta tagcgtgatc 40
<210> 40
<211> 57
<212> DNA
<213> Artificial Sequence
<400> 40
tcacgacgtt gtaaaacgac ggccagtgcc aagcttcacg gtcgggattt ctaacag 57
<210> 41
<211> 57
<212> DNA
<213> Artificial Sequence
<400> 41
catgattacg aattcgagct cggtacccgg ggatccagta gacacctcga acgctac 57
<210> 42
<211> 42
<212> DNA
<213> Artificial Sequence
<400> 42
gatcacgcta tagttgcgcc gaaaaggagc ttgctttacg ac 42
<210> 43
<211> 42
<212> DNA
<213> Artificial Sequence
<400> 43
gtcgtaaagc aagctccttt tcggcgcaac tatagcgtga tc 42
<210> 44
<211> 57
<212> DNA
<213> Artificial Sequence
<400> 44
tcacgacgtt gtaaaacgac ggccagtgcc aagcttcacg gtcgggattt ctaacag 57
<210> 45
<211> 52
<212> DNA
<213> Artificial Sequence
<400> 45
cgagctcggt acccggggat ccacccgggt gtggcgcgca agaagatgcc ag 52
<210> 46
<211> 46
<212> DNA
<213> Artificial Sequence
<400> 46
taaatgttgt acgcggacca gaacaagatt ccgccgtgga ccacgc 46
<210> 47

<211> 43
<212> DNA
<213> Artificial Sequence
<400> 47
ggcggaatct tgttctggtc cgcgtacaac atttacatac acc 43
<210> 48
<211> 51
<212> DNA
<213> Artificial Sequence
<400> 48
gtaaaacgac ggccagtgcc aagcttagca aggtgttaga gcaaattttc g 51
<210> 49
<211> 47
<212> DNA
<213> Artificial Sequence
<400> 49
gaattcgagc tcggtacccg gggatccaac ttgtaaccgc tccgtat 47
<210> 50
<211> 48
<212> DNA
<213> Artificial Sequence
<400> 50
ggtgtatgta aatgttgtac gcggaccagt gcttgccttg cttgtcta 48
<210> 51
<211> 48
<212> DNA
<213> Artificial Sequence
<400> 51
tagacaagca aggcaagcac tggtccgcgt acaacattta catacacc 48